# EUROPEAN PATENT APPLICATION

(11) **EP 4 756 834 A1**
(43) Date of publication of application: **10.06.2026**
(21) Application number: 24848090.7
(22) Date of filing: 23.07.2024
(51) Int. Cl.: G16H 40/67

(54) **BLOOD GLUCOSE MANAGEMENT SYSTEM FOR PROVIDING DATA INTERCOMMUNICATION**

(30) Priority: 31.07.2023 WO PCT/CN2023/110187
(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2024/106999
(87) International publication number: WO 2025/026132

(57) **Abstract**

The invention discloses a blood glucose management system that provides data exchange, comprising a blood glucose management system in the hospital and a blood glucose management system outside the hospital. The central server of the blood glucose management system in the hospital and the central server of the blood glucose management system outside the hospital are established in a communication connection, which can complement each other's missing blood glucose history data. During and after hospitalization, medical staff, patients themselves, guardians, or doctors outside the hospital can obtain complete blood glucose history records, facilitating the development of fast and effective treatment and rehabilitation plans.

## Description

### TECHNICAL FIELD

The invention mainly relates to the field of diabetes monitoring and management, and in particular, to a blood glucose management system that provides data exchange in and outside the hospital.

### BACKGROUND

The pancreas of healthy people can automatically secrete the required insulin/glucagon according to the blood glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods of continuously monitoring blood glucose are based on in vivo CGMs, which use disposable transdermal sensors inserted into the skin to measure blood glucose concentrations in interstitial fluids and send the blood glucose data through a transmitter to the remote device in real-time for the user to view. This monitoring method is called Continuous Blood glucose Monitoring (CGM).

General diabetes patients can achieve self-monitoring and management of blood glucose through continuous blood glucose monitoring, while diabetes patients with serious disease need to be hospitalized. Through the intervention of doctors, targeted treatment plans are taken to restore the blood glucose level of diabetes patients to a relatively stable state. At the same time, personalized medication, diet and exercise programs are provided to facilitate patients to achieve self-monitoring and management of blood glucose after discharge.

The blood glucose management system currently used in the hospital and the personal blood glucose management system used outside the hospital cannot communicate. After the patient is hospitalized, the medical staff cannot obtain the blood glucose historical data of the patient before hospitalization, and cannot quickly develop the personalized treatment plan for the patient. After the patient is discharged, the patient, the guardian or the doctor cannot obtain the blood glucose historical records of the patient during hospitalization, and cannot develop a fast and effective rehabilitation plan for the patient. This is adverse to the treatment of diabetes for the patient, and may even delay the treatment of the patient.

Therefore, there is an urgent need for a blood glucose management system that enables data exchange in and outside the hospital, based on existing technology.

### BRIEF SUMMARY OF THE INVENTION

The invention discloses a blood glucose management system that provides data exchange, allowing patients' blood glucose history data to be fully displayed to patients, medical staff, guardian, or attending doctors during hospitalization and after discharge, facilitating rapid and continuous treatment.

The embodiment discloses a blood glucose management system, comprising a blood glucose management system in the hospital and a blood glucose management system outside the hospital. The blood glucose management system in the hospital should at least include: A continuous blood glucose monitoring (CGM) device for continuously obtaining patients' blood glucose data. A receiver group comprising at least one receiver for receiving blood glucose data transmitted by CGMs in the hospital. A central server in the hospital is used to store the blood glucose data. A device of medical staff is used to manage patients' blood glucose data by medical staff. The blood glucose management system outside the hospital includes: A continuous blood glucose monitoring (CGM) device. A personal diabetes manager (PDM) device is used to manage the blood glucose data of patient. A central server outside the hospital is used to store blood glucose data. A communication connection between the central server in the hospital and the central server outside the hospital is established.

According to one aspect of the invention, the central server in the hospital interacts with the central server outside the hospital for one-way or two-way data exchange.

According to one aspect of the invention, the blood glucose data of the central server in the hospital and the central server outside the hospital are synchronously stored or read in real-time.

According to one aspect of the invention, the blood glucose management system outside the hospital further comprises a device of guardian for guardian and doctors out of hospital to manage patients' blood glucose data.

According to one aspect of the invention, a communication connection between the device of guardian and the central server outside the hospital or/and PDM is established.

According to one aspect of the invention, the blood glucose management system in the hospital further comprises a PDM, which establishes a communication connection with the central server in the hospital.

According to one aspect of the invention, the PDM verifies the patient's personal information before data exchange with the central server in the hospital.

According to one aspect of the invention, it further comprises a private server, which establishes a communication connection with at least one of the central server in the hospital and the central server outside the hospital.

According to one aspect of the invention, the device of medical staff or PDM can access the private server.

According to one aspect of the invention, the private server is used as a backup server for the central server in the hospital or the central server outside the hospital.

According to one aspect of the invention, the CGM establishes a communication connection with the receiver group or the PDM.

According to one aspect of the invention, the CGM switches to establish a communication connection between the receiver group and the PDM.

According to one aspect of the invention, the device of medical staff establishes a communication connection with the central server outside the hospital.

According to one aspect of the invention, the communication connection method comprises at least one of the following: the Internet, cellular data, or fiber optic.

Compared with the prior art, the technical solution of the invention has the following advantages:
The disclosed blood glucose management system provides data exchange, including a blood glucose management system in the hospital and a blood glucose management system outside the hospital. A communication connection between the central server of the blood glucose management system in the hospital and the central server of the blood glucose management system outside the hospital is established, which can complement each other's missing blood glucose history data. During and after hospitalization, medical staff, patients themselves, guardians, or doctors outside the hospital can obtain complete blood glucose history records, facilitating the development of fast and effective treatment and rehabilitation plans.

Further, after the patient is hospitalized, the PDM can be connected to the blood glucose management system in the hospital and establish a communication connection with the central server in the hospital. On the one hand, the PDM can serve as a backup receiver. When the receiver group in the hospital fails, the central server in the hospital can receive the patient's blood glucose data through the PDM to avoid data loss. On the other hand, after establishing a communication connection with the receiver group in the hospital, the CGM can no longer communicate with the PDM. At this time, the PDM can obtain data from the central server in the hospital, and the patient can still query their own blood glucose information to meet their usage needs.

Further, patients can also connect the private server to the blood glucose management system outside the hospital and the blood glucose management system in the hospital, and store their blood glucose data on the private server. Patients, medical staff, or guardian can access the private server on their respective devices to obtain the patient's blood glucose history data, which facilitates the development of fast and effective treatment and rehabilitation plans.

Further, the private server can be used as backup servers for the central servers in the hospital or the central servers outside the hospital. When the central server in the hospital or the central server outside the hospital go down, they can replace the access to the blood glucose management system in the hospital or the blood glucose management system outside the hospital, avoiding prolonged suspension of the blood glucose management system.

### BRIEF DESCRIPTION OF THE DRAWINGS

FIG.1 is a topological diagram of the blood glucose management system used in hospitals according to one embodiment of the invention.
FIG.2 is a schematic diagram of the integrated continuous CGM according to an embodiment of the invention.
FIG.3 is a schematic diagram of the split continuous CGM according to an embodiment of the invention.
FIG.4 is a comparison diagram of information stored in the central server according to an embodiment of the invention.
FIG.5 is an interface diagram of all the diabetic patient's blood glucose blood data information showed on the monitor screen according to an embodiment of the invention
FIG.6 is an interface diagram of a single diabetic patient's blood glucose blood data information showed on the monitor screen according to another embodiment of the invention.
FIG.7a to FIG.7c are schematic diagrams of a blood glucose management system for data exchange between internal and external hospitals according to embodiments of the invention.

### DETAILED DESCRIPTION

As mentioned above, the data of the blood glucose management system currently used in the hospital and the personal blood glucose management system used outside the hospital cannot be exchanged. After the patient is hospitalized, the medical staff cannot obtain the blood glucose historical data of the patient before hospitalization, and cannot quickly develop the personalized treatment plan for the patient. After the patient is discharged, the guardians or doctors visiting outside the hospital cannot obtain the blood glucose historical records of the patient during hospitalization, and cannot develop a fast and effective rehabilitation plan for the patient. This is detrimental to the treatment of diabetes for the patient, and even will delay the treatment of the patient.

In order to solve this problem, the invention discloses a blood glucose management system that provides data exchange, comprising a blood glucose management system in the hospital and a blood glucose management system outside the hospital. The central server of the blood glucose management system in the hospital and the central server of the blood glucose management system outside the hospital are established in a communication connection, which can complement each other's missing blood glucose history data. During and after hospitalization, medical staff, patients themselves, guardians, or doctors outside the hospital can obtain complete blood glucose history records, facilitating the development of fast and effective treatment and rehabilitation plans.

Various exemplary embodiments of the invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other parts.

The following description of the exemplary embodiments is merely illustrative, and is not intended to be in any way limiting the invention and its application or use. The techniques, methods, and devices that are known to those of ordinary skill in the art may not be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

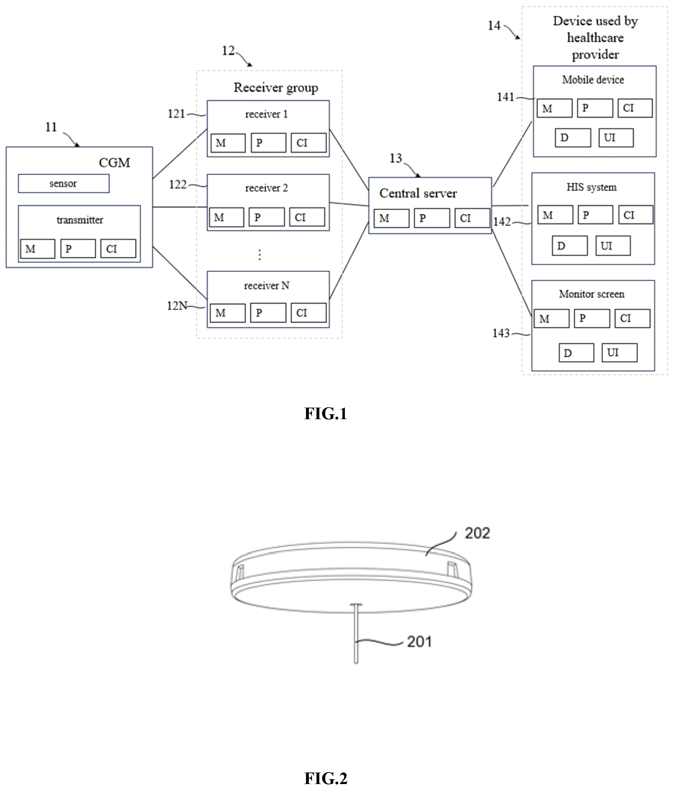

FIG.1 is a topological diagram of the blood glucose management system used in hospitals according to the embodiment of the invention.

In embodiments of the invention, a blood glucose management system used in a hospital includes: a CGM 11, used for monitoring of the patient's blood glucose level in real-time, mounted on the patient by means of an auxiliary applicator, comprising implantable sensors, the sensors being connected to a transmitter, the transmitter further comprising a memory (M), a processor (P), a communication interface (CI), and the like, and the transmitter is used for transmitting the blood glucose data information monitored by the CGM, as well as an identifier of the CGM 11, and the like. A receiver group 12 including a plurality of receivers, any one of the plurality of receivers can be used to receive and relay information transmitted by the transmitter, can be distributed in various locations in a hospital such as a ward, a doctor's office, a monitoring room, an operating room, a lavatory, and so on, as needed, and used to receive various types of data information from the CGM 11 such as information about the patient's blood glucose data, identifier of the CGM 11, and so on, and the receivers include a memory (M), a processor (P), a communication interface (CI), and so on, and can also send the various types of data information received from the transmitter to other devices. A central server 13, data center or cloud, hereinafter collectively referred to as the central server 13, for storing various types of data and information, including a memory (M), a processor (P), a communication interface (CI), etc., and can receive and store various types of data and information from various receivers, and can also communicate with an external device, which can be a receiver or a device used by healthcare provider 14, etc. The device used by healthcare provider 14, such as an operating system-containing mobile device 141 for interacting with the central server 13, a hospital information system (HIS system) 142 for viewing or deleting reports, a monitoring screen 143 for displaying blood glucose information for all patients, and so on, may include a memory (M), a processor (P), a communication interface (CI), a displayer (D), a user interface (UI), and so on. The CGM 11 and the plurality of receivers of the receiver group 12, the receiver group 12 and the central server 13, the central server 13 and the various devices 14 used by the healthcare providers are communicated wirelessly. Wireless communications may include but are not limited to, for example, radio frequency (RF) communications (e.g., radio frequency identification (RFID), Zigbee communication protocols, WiFi, infrared, wireless Universal Serial Bus (USB), Ultra-Wide Band (UWB), Bluetooth^{®} communication protocols, and cellular communications, such as Code Division Multiple Access (CDMA) or the Global System for Mobile Communications (GSM). Preferably, the CGM 11 and the plurality of receivers of the receiver group 12, the receiver group 12 and the central server 13, the central server 13 and the various healthcare provider's end-user devices 14 are communicated by Wifi and/or cellular communications.

No matter where the patient is located in the hospital, the data transmitted by the transmitter will be received by the receiver that is at the optimal communication distance, avoiding that all the receivers or a plurality of receivers of the receiver group 12 receive the data, on the one hand, it can save the power consumption of the transmitter and, on the other hand, it can prevent each receiver from receiving the data and then uploading the data to the centralized server, which results in the duplication of the blood glucose data information and occupies the storage space of the central server.

The CGM 11 is a wearable continuous CGM (hereinafter referred to as CGM), the CGM comprising a sensor and a transmitter, the sensor being used to collect analyte data in the human body and transmit the collected analyte content information, i.e., the sensor is used to collect information on blood glucose data in the human body and transmit the information. The transmitter is connected to the sensor, and it is used to receive the information on the blood glucose data, which is transmitted by the sensor that has been implanted under the skin, and convert the information into a wireless signal for outputting, and at the same time, it can also output other information such as information on an identifier of the CGM. Each CGM has a unique identifier including, but not limited to a device identifier, a hardware identifier, a generic unique identifier, a serial number, an identifier based on communication protocol (e.g., a BLE ID), a manufacturer's identifier, and the like, preferably, identifier information is the serial number of the CGM, which is formed by a plurality of randomly-combined numerical and alphabetic, and can be set on a housing or package of the CGM, or may also be set differently depending on the type of the CGM.

Specifically, in one embodiment of the invention, the CGM is integrated, i.e., the sensor and the transmitter have been integrated together prior to use, and is a single-use product, which is discarded after use, as shown in FIG. 2. FIG.2 is a schematic diagram of the integrated continuous CGM according to an embodiment of the invention. The integrated continuous CGM includes a sensor 201, a housing 202, and a transmitter (not shown in the figure) set inside the housing 202, the sensor 301 is used to monitor the patient's body fluid blood glucose data information, and transmit the said blood glucose data information to the transmitter through an internal circuit, which in turn sends it to a receiver. The identifier may be provided on the outer housing of the CGM or on the outer packaging or within the CGM.

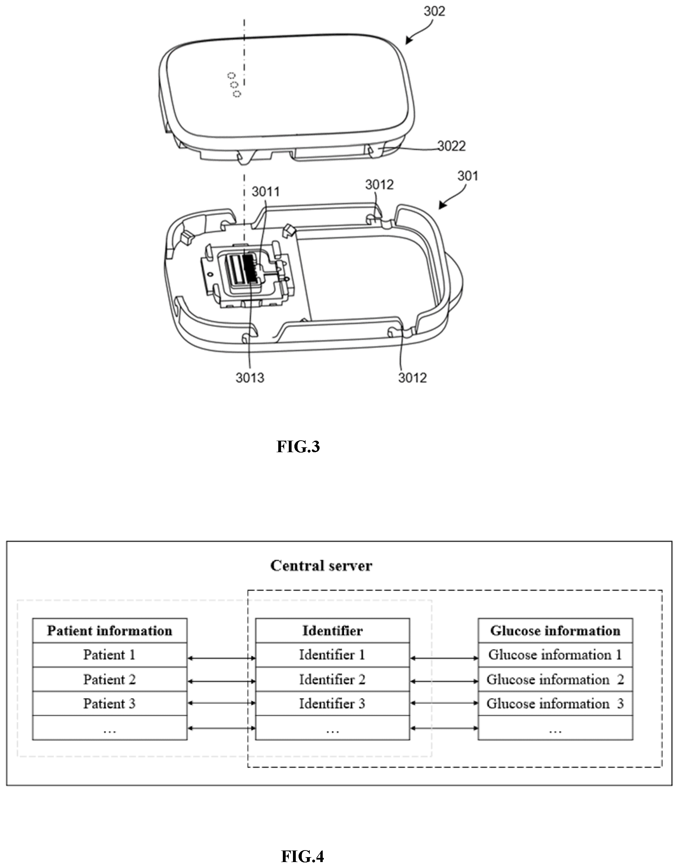

In another embodiment of the invention, the CGM is split, i.e., the sensor and the transmitter are two different parts prior to use, packaged separately, and only integrated together when in use, as shown in FIG. 3. FIG.3 is a schematic diagram of the split continuous CGM according to an embodiment of the invention. The split continuous CGM includes a base 301 and a transmitter 302, the base is provided with a sensor 3011, the transmitter 302 has a separate housing, the housings of the base 301 and the transmitter 302 are respectively provided with snap-in structures 3012 and 3022, the base 301 and the transmitter 302 are snapped into a whole through the snap-in structures when in use, the sensor 3011 is electrically connected to the transmitter 302 through the electrical connection 3013, the sensor 301 is used to monitor the patient's blood glucose data information, the blood glucose data information is transmitted to the transmitter 302 through the electrical connection 3013, and then sent to the receiver by the transmitter 302.

In one embodiment of the invention, both the sensor and the transmitter of the split continuous CGM are single-use products, which are discarded after use, and therefore the identifier may be provided on the housing or the outer packaging of the sensor or the transmitter. In yet another embodiment of the invention, only the sensor of the split continuous CGM is a single-use product and the transmitter is a reusable product, therefore, preferably, in this embodiment, the identifier is provided on the housing or the outer packaging of the transmitter, which reduces the frequency of tying the patient's information to the identifier and improves the patient experience. This will be described in more detail below.

When the identifier is set on the housing or outer packaging of the CGM or transmitter, it is set in a form that includes, but is not limited to, a QR code, a bar code, or an NFC tag.

When a patient is admitted to the hospital, the healthcare provider creates a new patient account via the mobile device 141, matches the patient's personal information with the identifier of the worn CGM, and uploads the patient's personal information and the corresponding identifier to the central server 13, where the patient's personal information includes name, age, gender, department, bed number, ward, diagnosis, inpatient hospitalization number, cell phone number, and so on. When the CGM worn by the patient needs to be replaced with a new CGM due to reaching its life cycle or failure, etc., the unique identifier information of the new CGM also needs to be updated by pairing with the patient's personal information through the mobile device 141 and further uploaded to the central server 13. The central server 13 stores the patient's personal information and corresponding identifier information. The patient's personal information can be entered manually or by scanning the patient's inpatient bracelet to automatically match the relevant information, and the identifier information can also be entered manually or by scanning the QR code, bar code, or NFC tag on the CGM housing or the outer packaging.

When the CGM is a split CGM and the transmitter is reusable, the CGM identifier is set on the transmitter's housing or packaging, so that when the patient needs to replace the CGM, he or she only needs to replace the sensor, not the transmitter, and the CGM identifier remains unchanged. As a result, there is no need to update the pairing update of the CGM identifier with the patient's personal information via the mobile device 141, and even less need for further uploading to the central server 13, thus reducing the number of operational steps and improving the patient experience.

After the CGM has being worn on the patient and started working, the CGM sends a wireless signal after completing each blood glucose monitoring, and preferably, the wireless signal sent by the CGM is a Bluetooth signal, as previously described. Theoretically, each receiver of the receiver group 12 can receive the Bluetooth signal sent by the CGM, but after the Bluetooth signal sent by the CGM is received by the receiver that is within the optimal communication distance, the transmitter stops transmitting the blood glucose data signal for this blood glucose monitoring circle, which on the one hand, saves the power consumption of the transmitter, and on the other hand, it can prevent that each receiver receives the data and uploads the data to the central server, which results in the duplication of the blood glucose data information, and occupies the storage space of the central server. The Bluetooth signal contains the CGM's current blood glucose monitoring data information and the corresponding CGM identifier information. A receiver located within an optimal communication distance receives the CGM's current blood glucose data information and the corresponding CGM identifier information, which is further uploaded to the central server 13. When the patient is in a different position, the current blood glucose data information and the corresponding CGM identifier information sent by the transmitter will be received by different receivers located in the optimal communication position compared to the patient's position, and the different receivers will upload the current blood glucose data information and the corresponding CGM identifier information sent by the CGM to the central server 13 after receiving them. That is, the central server 13 may store all of the blood glucose data information and corresponding identifier information received from the different receivers.

Therefore, the corresponding blood glucose data information and the identifier information, the corresponding patient personal information and the identifier information are stored in the storage module of the central server 13 at the same time. Generally, the corresponding blood glucose data information and the identifier information, the corresponding patient personal information and the identifier information are stored in different storage modules of the central server 13, but the two sets of data are related to each other, and the pairing of the patient personal information and the blood glucose data information can be accomplished by the identifier information, as shown in FIG. 4.

As mentioned earlier, when a patient is admitted to the hospital, the healthcare provider will create a new patient account through the mobile device 141 to realize the function of patient management, in addition to account management, device management, blood glucose data information viewing and report printing, and so on, specifically:
In patient management, after clicking Add Account, you can scan the patient's bracelet or manually enter the patient's personal information, and at the same time scan or manually enter the CGM identifier information, and through the mobile phone authentication code to verify the patient's personal information and add the patient account, and when it is necessary to delete the patient account, clicking Delete to delete the corresponding account.

In account management, the mobile device 141 is set up with one or more processors that enable multiple individuals with different access level rights to modify the setup parameters of the mobile device. The supervising physician can create an HCP master account, and the HCP master account can create HCP sub-accounts to add and view all information about all patients, view the operation records of the HCP master account and the HCP sub-accounts, and the like. And the supervising physician with the HCP master account can manage hospital-wide HCP sub-accounts, departments and patients information, with read/write privileges to HCP sub-accounts, departments, and also assigns access privileges to HCP sub-accounts, e.g., a resident doctor with a HCP sub-account is assigned with editing privileges to HCP sub-accounts to modify the patient information in the department, add a patient information, and view all the patients' information, while a nurse with a HCP sub-account is assigned with privileges to HCP sub-accounts to view all the patient information in the department only.

In device management, mobile device 141 can view the status of all connected transmitters and receivers, and can add or remove transmitters and receivers.

The mobile device 141 can also set blood glucose alarms, including global blood glucose alarms and individual blood glucose alarms, the global blood glucose alarms is a default blood glucose alarms for all patients, including a high blood glucose threshold, e.g., 13.3 mmol/L. and a low blood glucose threshold, e.g., 4.4 mmol/L. a predictive blood glucose alarms, such as a rise in blood glucose values to 13.3 mmol/L or even 22.2 mmol/L in the coming period of time, e.g., half an hour, one hour, or a fall in blood glucose values to 4.4 mmol/L or even 2.2 mmol/L in the coming period of time. an alarm on the rate of change of blood glucose, such as rapid rise/rapid fall of blood glucose at a rate of 0.110 mmol/L/min , or even 0.170 mmol/L/min. a hypoglycemic alarm, e.g., blood glucose below 3.1 mmol/L. The global blood glucose alarm means that all patients are set to the same blood glucose high/low threshold, and once any patient's blood glucose value is monitored to reach the set threshold, the mobile device 141 will active an alarm. According to different settings, the alarms can be set as one or a combination of sound alarms, vibration alarms, and interface text alarms. The sound type, volume size, duration of the sound, frequency interval of the sound alarm. the vibration type, intensity of the vibration, duration of the vibration, frequency interval of the vibration alarm. the type of the interface text alarm, the frequency of the alarm, etc. can be personalized according to the actual needs or the patient's preferences, or refer to the contents disclosed in patent PCT/ CN2022/CN119084.

In general, the blood glucose alarm settings for new patients are defaulted to match the global alarm settings. If a patient's blood glucose alarm threshold to be set can not match the global blood glucose alarm threshold due to his/her own constitution or at a special stage, such as pregnancy, or due to other illnesses, specific individual blood glucose alarms can be set in the patient management page.

When the healthcare provider needs to view the patient's blood glucose monitoring data or prints a report, he or she enters the patient's information into the mobile device 141 or the operating system of the HIS system 142, and by the identifier and the patient's correspondence, the corresponding patient's blood glucose data information can be located in the data memory, and displayed on the monitor.

When viewing the blood glucose data information via the mobile device 141 the current blood glucose data information and the blood glucose trend of the patient can be displayed on the real-time monitoring page of the patient management, and the blood glucose data information in a recent period of time is displayed in the form of a curve, such as three hours, six hours, nine hours, twelve hours, twenty-four hours, or forty-eight hours, and so on, and the blood glucose monitoring trend graph of the corresponding period of time is displayed by switching the option of different periods of time. Similarly, when you need to print a report, you can select the desired report time and report type on the report print page of patient management, and then print the report, and the electronic copy of the blood glucose report is automatically saved to the mobile device 141. The types of reports include a comprehensive report on blood glucose monitoring, an ambulatory blood glucose (AGP) chart, and a daily detail chart. The comprehensive report includes daily statistics and overall statistics of blood glucose values measured during the monitoring cycle. Statistical parameters include: number of points measured. mean value. standard deviation. coefficient of variation. average fluctuation. maximum/minimum blood glucose value. distribution of blood glucose, etc. AGP Chart displays the blood glucose AGP profile and blood glucose distribution histogram during the monitoring cycle. The following statistics are provided: CGM usage time proportion, mean value, standard deviation, coefficient of variation, estimated glycated hemoglobin, average fluctuation, average day-to-day variation, and number of high/low blood glucose events per day. The daily detail chart displays a graph of glycemic trends and simple statistics for each day of the monitoring cycle.

HIS system is a digital and networked management system for the various operations and medical activities of each department of the hospital, which involves all information management, including administrative management system, medical management system, decision support system, and various other auxiliary systems. The medical management system is mainly involved in the hospital's medical business information processing, mainly including outpatient, emergency management system, case management system, medical statistics query system, blood bank management system and so on. In the medical management system, you can search, delete, view and print all kinds of blood glucose data information reports of patients.

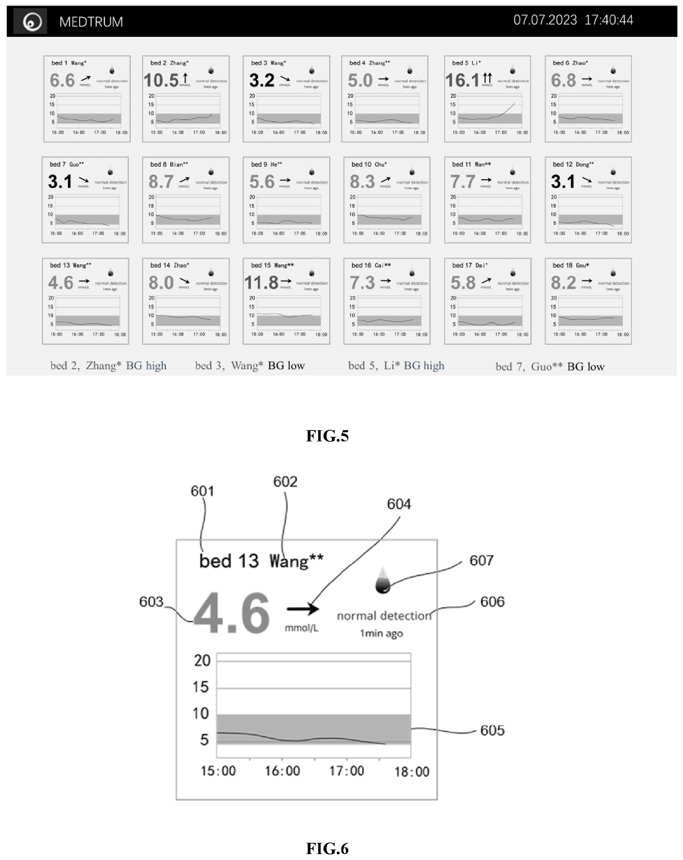

The monitoring screen 143 used at the nurses' station displays blood glucose data information for all monitored patients, as shown in Fig. 5, and also highlights information about patients with abnormal blood glucose data, such as bed number, name, and abnormal blood glucose data information. Abnormal blood glucose readings are highlighted in different colors, e.g., high blood glucose is displayed in yellow, low blood glucose in red, and the patient's abnormal blood glucose status is displayed individually in the form of text on the monitoring screen 143. Each patient's blood glucose monitoring data information is shown in Fig.6, including bed number 601, name 602, recent blood glucose reading 603, blood glucose trend arrow 604, blood glucose trend graph 605, sensor status 606, and calibration icon 607. Through the monitoring screen 143, healthcare providers, such as nurses, can quickly and clearly understand the status of all the patient's blood glucose monitoring, and the existence of any accidental or unexpected situation can be dealt with or feedback to the doctor in time.

The mobile device 141, the HIS system 142, and the monitoring screen 143 are different display devices that may provide different user interfaces, and the content of the interface displays, such as the amount, format and/or type of data to be displayed, alarms, and the like, may be customized by the manufacturer and/or the end user for each particular display device.

In another embodiment of the invention, a personal diabetes management device may also be configured for a patient when the patient is in a hospital, i.e., the blood glucose management device used in the hospital may also include a personal diabetes management device, wherein the personal diabetes management device is matched with the CGM by the identifier information of the CGM used by the patient and communicates wirelessly. Wireless communication can be via, for example, but not limited to, radio frequency (RF) communication (e.g., radio frequency identification (RFID), Zigbee communication protocols, WiFi, infrared, wireless Universal Serial Bus (USB), Ultra-Wide Band (UWB), Bluetooth^{®} communication protocols, and cellular communication, such as Code Division Multiple Access (CDMA) or Global System for Mobile Communications (GSM). Preferably, the personal diabetes management device communicates with the CGM via Bluetooth, facilitating the patient to know his/her blood glucose information in the first instance, and at the same time to learn and become proficient in the use of the personal diabetes management device, facilitating the use of the management device by himself/herself to monitor and manage his/her blood glucose level after discharged from the hospital. When the patient is an elderly person or a young child, or a diabetic patient with a special disease who is unable to conduct self-monitoring of blood glucose, healthcare professionals can limit the patient's operation on the personal diabetes management device through the lock mode on the mobile device 141, so that the patient is unable to view the real-time blood glucose data information, receive blood glucose alarms, or change the alarm settings on the personal diabetes management device, etc.. Therefore, it can prevent the misuse of the personal diabetes management device by the patient, such as deleting the device, releasing the connection relationship between the personal diabetes management device and the CGM, which may affect the normal blood glucose monitoring of the CGM. not accepting the blood glucose alarm also prevents the blood glucose alarm from interfering with or affecting the patient.

In other embodiments of the invention, a blood glucose management device for use in the hospital may further comprise a secondary management device, the secondary management device also being matched to the CGM via identifier information of the CGM used by the patient, and communicating wirelessly. Wireless communication can be via, for example, but not limited to, radio frequency (RF) communication (e.g., radio frequency identification (RFID), Zigbee communication protocols, WiFi, infrared, wireless Universal Serial Bus (USB), Ultra-Wide Band (UWB), Bluetooth^{®} communication protocols, and cellular communication, such as Code Division Multiple Access (CDMA) or Global System for Mobile Communications (GSM). Preferably, the secondary management device communicates with the CGM via Bluetooth. Although the patient can perform blood glucose monitoring by himself or herself, the guardian or healthcare provider may also use the secondary management device in order to keep track of the patient's blood glucose level, at that time the content displayed by the secondary management device is the same as the content displayed by the personal diabetes management device. When the user of the secondary management device is a healthcare provider, the secondary management device and the mobile device 141 of the healthcare provider's end-user device can be the same device.

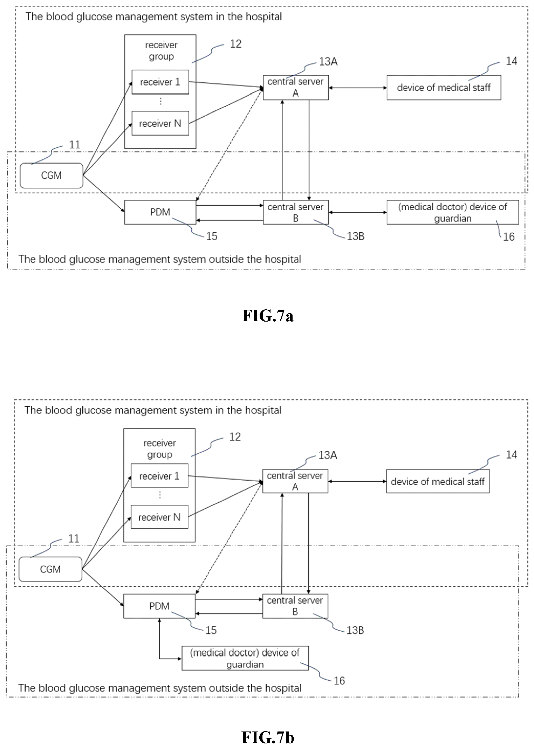

Figs. 7a to 7c are schematic diagrams of a blood glucose management system for data exchange between internal and external hospital.

Referring to Fig. 7a, in the embodiment of the invention, the blood glucose management system outside the hospital includes at least one CGM 11 for real-time monitoring of patient blood glucose data and sending these blood glucose data to the outside world. The PDM 15, such as smart phone, personal diabetes manager (PDM) or computer, can be pre-installed or downloaded with blood glucose management software to receive and process blood glucose data information sent by CGM 11. The central server 13B outside the hospital is a data storage and transfer server established by the manufacturer of CGM 11 for patients. Patients establish communication with the server through their PDM 15 to manage their own blood glucose data. The central server 13B outside the hospital needs to strictly keep patient data confidential.

In some embodiments of the invention, the blood glucose management system outside the hospital may further include device of guardian or doctor, hereinafter referred to as device of guardian 16, which can access the central server 13B for assisting in managing patients' blood glucose data outside the hospital.

In some embodiments of the invention, a communication connection is established between the central server 13A and the central server 13B, for example, through the Internet, cellular data, fiber optics, etc., to achieve data exchange between the central server 13A in the hospital and the central server 13B outside the hospital, so that medical staff can access the central server 13B outside the hospital through the central server 13A and obtain blood glucose data of patients. Of course, patients, guardians, and doctors outside the hospital can also access the central server 13A in the hospital through the central server 13B and obtain the patient's blood glucose data.

In some embodiments of the invention, the data exchange between the central servers 13A and 13B can also be one-way. For example, medical staff can access the central server 13B outside the hospital through the central server 13A in the hospital to view the patient's blood glucose history records. However, at the same time, guardian and doctors outside the hospital cannot access the central server 13A in the hospital through the central server 13B outside the hospital. This is to consider the need for confidentiality of the patient's personal data when receiving treatment in the hospital. On the contrary, after the patient is discharged, the guardian and doctors outside the hospital can access the central server 13A in the hospital through the central server 13B outside the hospital. However, at the same time, medical staff cannot access the central server 13B outside the hospital through the central server 13A in the hospital. This is to consider the need for patients to keep their personal data confidential after discharge.

Referring to Fig. 7b, in some embodiments of the invention, the device of guardian 16 can establish a communication connection with the PDM 15, and exchange data with it. It can also be used to assist in managing patients' blood glucose data outside the hospital.

In some embodiments of the invention, the device of guardian 16 can replace the PDM 15 to establish a communication connection with the central server 13B outside the hospital. The blood glucose data obtained by the CGM 11 is transmitted to the device of guardian 16 through the personal management device 15, and then transmitted to the central server 13B outside the hospital.

In some embodiments of the invention, the guardian can establish communication connections with both the PDM 15 and the central server 13B outside the hospital using the device of guardian 16. Blood glucose data can be transmitted through the signal channel selected by the patient or the default channel with better signal quality.

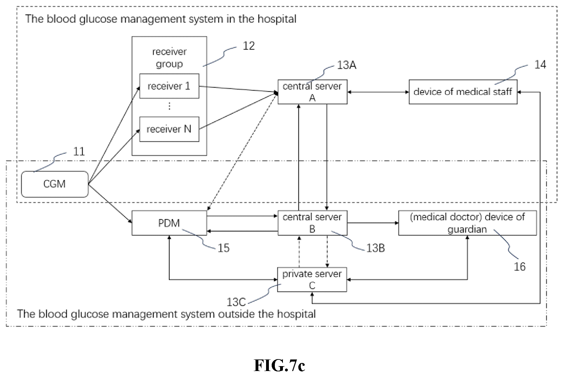

Referring to Fig. 7c, in some embodiments of the invention, patients can also establish their own private server 13C and store blood glucose data on the private server 13C. Patients can authorize guardian to access the server using device of guardian 16, or authorize device of medical staff 14 to access the private server 13C. The private server 13C can be a cloud server or a local server on the patient's personal computer.

According to the embodiments illustrated in Figs. 7a to 7c, both blood glucose management system in the hospital and blood glucose management system outside the hospital can achieve blood glucose data exchange of patients between inside and outside the hospital. The specific usage method will be described in detail in the following text.

In the existing technology, after establishing a communication connection with the receiver group 12, the CGM 11 cannot establish a communication connection with the PDM 15, which will result in patients being unable to read blood glucose data through the PDM 15 after hospitalization. Considering such usage issues, in some embodiments of the invention, the PDM 15 can also access the central server 13A in the hospital and exchange data with the central server 13A in the hospital. After the CGM 11 of the patient transmits the blood glucose data to the central server 13A through the receiver group 12, the patient can read the blood glucose data through the PDM 15 to meet the patient's in-hospital usage needs.

In some embodiments of the invention, before the patient is hospitalized, their blood glucose data is stored in the central server 13B outside the hospital, and after the patient is hospitalized, medical staff enter the patient's personal information, such as the patient's mobile phone number, name, SN (Serial Number) code of the CGM 11, and other information into the blood glucose management system in the hospital. The central server 13A in the hospital can establish a communication connection with the central server 13B outside the hospital, or the central server 13A in the hospital can apply to establish a communication connection with the central server 13B outside the hospital, and after confirmation by the manufacturer of the CGM 11, establish a communication connection.

In some embodiments of the invention, after establishing a communication connection between the central server 13A in the hospital and the central server 13B outside the hospital, patients or medical staff can operate the blood glucose management system in the hospital to read the patient's blood glucose history data before hospitalization, and synchronize and store these blood glucose history data in the central server 13A in the hospital. Afterwards, medical staff can read the patient's blood glucose history data at any time to facilitate the development of treatment plans for the patient.

In other embodiments of the invention, the blood glucose history data of patients before hospitalization does not need to be stored in the central server 13A in the hospital. Medical staff can read the blood glucose history data stored in the central server 13B outside the hospital in real time through the network.

After the patient is hospitalized, the receiver group 12 in the hospital may malfunction and cannot receive real-time blood glucose data from the CGM 11. The central server 13A may have data loss, and device of medical staff 14 may also be unable to view the patient's blood glucose data in real time, affecting the patient's treatment. To avoid this problem, in some embodiments of the invention, the PDM 15 can also be used in the hospital as an alternative receiver, establishing a communication connection with the central server 13A and exchanging data with it. When the receiver group 12 malfunctions, the data from the CGM 11 is transferred through the PDM 15, transmitted and stored to the central server 13A, and device of medical staff 14 can still view the patient's blood glucose data in real time.

In other embodiments of the invention, after the receiver group 12 fails, the PDM 15 can continue to exchange data with the central server 13B outside the hospital, so the central server 13B outside the hospital can transmit and store blood glucose data to the central server 13A in the hospital, such device of medical staff 14 can still view the patient's blood glucose data in real time.

In general use, the central server 13A in the hospital prioritizes receiving blood glucose data from the receiver group 12. When the receiver group 12 fails, the central server 13A in the hospital will only receive blood glucose data from the PDM 15.

In some embodiments of the invention, when the central server 13A in the hospital receives blood glucose data from the PDM 15, it needs to first verify the patient's personal information. When a patient is hospitalized, it is necessary to input personal information such as the SN code of the CGM 11 and the patient's phone number into the device of medical staff 14. Similarly, when a patient uses a PDM, it is also necessary to input personal information such as the SN code of the CGM 11 and the patient's phone number. When the central server 13A in the hospital receives blood glucose data from the PDM 15, the central server 13A in the hospital or the device of medical staff 14 compares the patient's personal information. If the patient's information is the same, the central server 13A in the hospital receives the blood glucose data from the PDM 15. Otherwise, it prompts the patient or medical staff to check whether the information input is correct. At this time, the device of medical staff 14 will not display the patient's personal information.

In some embodiments of the invention, as mentioned earlier, after hospitalization, patients can connect to the central server 13A in the hospital through the PDM 15 to obtain blood glucose data. When the patient changes the CGM 11 during hospitalization, the SN code of the CGM 11 will be updated in the central server 13A. At this time, the PDM 15 still uses the old SN code, which will cause the PDM to be unable to receive data from the central server 13A. Therefore, patients need to update the SN code on the PDM 15 to maintain data interaction with the central server 13A.

In some embodiments of the invention, if a patient has pre entered their personal information on the device of medical staff 14, and the patient has already used the PDM 15 before hospitalization and entered the same personal information into the PDM 15, the CGM 11 can automatically switch to connect to the receiver group 12 in the hospital after hospitalization and connect to the blood glucose management system in the hospital, in order to facilitate the patient's admission treatment.

In some embodiments of the invention, patients may choose to store their personal blood glucose data in a private server 13C, which can establish a communication connection with the central server 13B outside the hospital or the central server 13A in the hospital and exchange data. Medical staff or doctors seeking medical treatment outside the hospital can access the private server 13C on their respective devices to read patients' blood glucose data.

In some embodiments of the invention, whether it is medical staff or doctors seeking medical treatment outside the hospital, confirmation from the patient is required before accessing the patient's private server 13C. Otherwise, access to the private server 13C is not possible.

In some embodiments of the invention, after establishing a communication connection with the central server 13B outside the hospital or the central server 13A in the hospital, the private server 13C can be used as a backup server for the central server 13B outside the hospital or the central server 13A in the hospital, and the patient's blood glucose data can be backed up in the private server 13C. When the central server 13B outside the hospital or the central server 13A in the hospital goes down, it can be used as a data storage and transfer server to access the blood glucose management system in the hospital or the blood glucose management system outside the hospital, avoiding prolonged suspension of the blood glucose management system and affecting patient treatment and rehabilitation.

In summary, the embodiments of the invention disclose a blood glucose management system that provides data exchange, comprising a blood glucose management system in the hospital and a blood glucose management system outside the hospital. The central server of the blood glucose management system in the hospital and the central server of the blood glucose management system outside the hospital are established in a communication connection, which can complement each other's missing blood glucose history data. During and after hospitalization, medical staff, patients themselves, guardians, or doctors outside the hospital can obtain complete blood glucose history records, facilitating the development of fast and effective treatment and rehabilitation plans.

While the invention has been described in detail with reference to the specific embodiments of the invention, it should be understood that it will be appreciated by those skilled in the art that the above embodiments may be modified without departing from the scope and spirit of the invention. The scope of the invention is defined by the appended claims.

## Claims

1. A blood glucose management system, comprising:
A blood glucose management system in the hospital and a blood glucose management system outside the hospital.
The blood glucose management system in the hospital should at least include: A continuous blood glucose monitoring (CGM) device for continuously obtaining patients' blood glucose data. A receiver group comprising at least one receiver for receiving blood glucose data transmitted by CGMs in the hospital. A central server in the hospital is used to store the blood glucose data. A device of medical staff is used to manage patients' blood glucose data by medical staff.
The blood glucose management system outside the hospital includes: A continuous blood glucose monitoring device. A personal diabetes manager device is used to manage the blood glucose data of patient. A central server outside the hospital is used to store blood glucose data.
Wherein, a communication connection between the central server in the hospital and the central server outside the hospital is established.

2. The blood glucose management system according to claim 1, **characterized in that**, the central server in the hospital interacts with the central server outside the hospital for one-way or two-way data exchange.

3. The blood glucose management system according to claim 1, **characterized in that**, the blood glucose data of the central server in the hospital and the central server outside the hospital are synchronously stored or read in real-time.

4. The blood glucose management system according to claim 1, **characterized in that**, the blood glucose management system outside the hospital further comprises a device of guardian for guardian and doctors out of hospital to manage patients' blood glucose data.

5. The blood glucose management system according to claim 4, **characterized in that**, a communication connection between the device of guardian and the central server outside the hospital or/and the PDM is established.

6. The blood glucose management system according to claim 1, **characterized in that**, the blood glucose management system in the hospital further comprises a PDM, which establishes a communication connection with the central server in the hospital.

7. The blood glucose management system according to claim 6, **characterized in that**, the PDM verifies the patient's personal information before data exchange with the central server in the hospital.

8. The blood glucose management system according to claim 1, **characterized in that**, it further comprises a private server, which establishes a communication connection with at least one of the central server in the hospital and a central server outside the hospital.

9. The blood glucose management system according to claim 8, **characterized in that**, the device of medical staff or PDM can access the private server.

10. The blood glucose management system according to claim 8, **characterized in that**, the private server is used as a backup server for the central server in the hospital or the central server outside the hospital.

11. The blood glucose management system according to claim 1, **characterized in that**, the CGM establishes a communication connection with the receiver group or the PDM.

12. The blood glucose management system according to claim 11, **characterized in that**, the CGM switches to establish a communication connection between the receiver group and the PDM.

13. The blood glucose management system according to claim 1, **characterized in that**, the device of medical staff establishes a communication connection with the central server outside the hospital.

14. The blood glucose management system according to claim 1, **characterized in that**, the communication connection method comprises at least one of the following: the Internet, cellular data, or fiber optic.
